# EUROPEAN PATENT APPLICATION

(11) **EP 3 800 216 A1**
(43) Date of publication of application: **07.04.2021**
(21) Application number: 20198861.5
(22) Date of filing: 29.09.2020
(51) Int. Cl.: C08G 65/336, C03C 17/30, C09D 171/02

(54) **HYDROPHILIC SUBSTRATE AND METHOD OF MANUFACTURING HYDROPHILIC SUBSTRATE**

(30) Priority: 02.10.2019 JP 2019182374
(71) Applicant: Sumitomo Rubber Industries, Ltd., Kobe-shi, Hyogo-ken 651-0072 (JP)
(72) Inventor: MINAGAWA, Yasuhisa, Kobe-shi, Hyogo 651-0072 (JP); TAKII, Mitsuko, Kobe-shi, Hyogo 651-0072 (JP)
(74) Representative: Manitz Finsterwald Patent- und Rechtsanwaltspartnerschaft mbB

(57) **Abstract**

Provided are a hydrophilic substrate provided with a highly smooth hydrophilic silane compound layer having less surface irregularities and a method of manufacturing the hydrophilic substrate. The present invention relates to a hydrophilic substrate including a substrate having a hydrophilized surface and a hydrophilic silane compound layer provided on at least part of the hydrophilized surface of the substrate.

## Description

### TECHNICAL FIELD

The present invention relates to a hydrophilic substrate including a hydrophilic silane compound layer provided on a hydrophilized surface of a substrate and a method of manufacturing the hydrophilic substrate.

### BACKGROUND ART

It has been proposed to coat the surface of substrates with special polymers in order to prepare devices for capturing specific cells (e.g., blood cells, cancer cells present in blood or biological fluid) from blood or biological fluid.

However, some special polymers hardly provide a smooth surface by coating. Since surface irregularities can affect the ability to capture specific cells, it is desirable to provide substrates having controlled surface irregularities which are excellent in properties such as the ability to capture specific cells such as cancer cells (see, for example, Patent Literature 1). Moreover, since capturing specific cells is usually performed in aqueous solutions, it is also desirable to provide substrates which exhibit controlled surface irregularities and smoothness in aqueous solutions.

### CITATION LIST

### PATENT LITERATURE

Patent Literature 1: JP 2005-523981 T

### SUMMARY OF INVENTION

### TECHNICAL PROBLEM

The present invention aims to solve the problem and provide a hydrophilic substrate provided with a highly smooth hydrophilic silane compound layer having less surface irregularities and a method of manufacturing the hydrophilic substrate.

### SOLUTION TO PROBLEM

The present invention relates to a hydrophilic substrate, including:
a substrate having a hydrophilized surface; and
a hydrophilic silane compound layer provided on at least part of the hydrophilized surface of the substrate.

In the hydrophilic substrate, the hydrophilization is preferably an alkaline solution treatment.

In the hydrophilic substrate, the hydrophilization is preferably at least one selected from the group consisting of ultraviolet irradiation, electron beam irradiation, ion irradiation, and plasma treatments.

In the hydrophilic substrate, the hydrophilic silane compound layer is preferably formed from at least one of a hydrophilic silane compound solution or a hydrophilic silane compound dispersion.

In the hydrophilic substrate, the hydrophilic silane compound layer is preferably formed by starting treatment with the at least one of a hydrophilic silane compound solution or a hydrophilic silane compound dispersion within three hours after the hydrophilization.

In the hydrophilic substrate, the hydrophilic silane compound layer is preferably formed from a hydrophilic silane compound represented by the following formula (I): 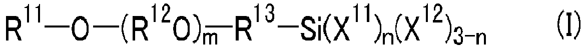 wherein R¹¹ represents a monovalent hydrocarbon group; R¹² and R¹³ are the same or different and each represent a divalent hydrocarbon group; each X¹¹ independently represents an alkoxy group; each X¹² independently represents a monovalent hydrocarbon group or a halogen group; m represents 1 to 50; and n represents 0 to 3.

In the hydrophilic substrate, the silane compound of formula (I) is preferably a compound represented by the following formula (I-1):

R²¹-O-(CₚH₂ₚO)ₘ-(CH₂)ₖ-Si(X¹¹)ₙ(X¹²)₃₋ₙ (I-1)

wherein R²¹ represents a methyl group or an ethyl group;
each X¹¹ independently represents an alkoxy group; each X¹² independently represents a monovalent hydrocarbon group or a halogen group; p represents 1 to 8; m represents 1 to 50;
n represents 0 to 3; and k represents 1 to 20.

In the hydrophilic substrate, the hydrophilic silane compound layer preferably has fibronectin adsorbed thereto.

In the hydrophilic substrate, the hydrophilic silane compound layer preferably has a thickness of 1 to 50 nm.

The present invention also relates to a method of manufacturing a hydrophilic substrate, including:
hydrophilizing a surface of a substrate; and
forming a hydrophilic silane compound layer on at least part of the hydrophilized surface.

In the method of manufacturing a hydrophilic substrate, the hydrophilization is preferably an alkaline solution treatment.

In the method of manufacturing a hydrophilic substrate, the hydrophilization is preferably at least one selected from the group consisting of ultraviolet irradiation, electron beam irradiation, ion irradiation, and plasma treatments.

In the method of manufacturing a hydrophilic substrate, the hydrophilic silane compound layer is preferably formed from at least one of a hydrophilic silane compound solution or a hydrophilic silane compound dispersion.

In the method of manufacturing a hydrophilic substrate, the hydrophilic silane compound layer is preferably formed by starting treatment with the at least one of a hydrophilic silane compound solution or a hydrophilic silane compound dispersion within three hours after the hydrophilization.

In the method of manufacturing a hydrophilic substrate, the hydrophilic silane compound layer is preferably formed from a hydrophilic silane compound represented by the following formula (I): 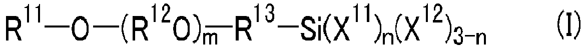 wherein R¹¹ represents a monovalent hydrocarbon group; R¹² and R¹³ are the same or different and each represent a divalent hydrocarbon group; each X¹¹ independently represents an alkoxy group; each X¹² independently represents a monovalent hydrocarbon group or a halogen group; m represents 1 to 50; and n represents 0 to 3.

In the method of manufacturing a hydrophilic substrate, the hydrophilic silane compound layer preferably has a thickness of 1 to 50 nm.

### ADVANTAGEOUS EFFECTS OF INVENTION

The hydrophilic substrate of the present invention includes a substrate having a hydrophilized surface and a hydrophilic silane compound layer provided on at least part of the hydrophilized surface of the substrate. Thus, the present invention provides a hydrophilic substrate provided with a highly smooth hydrophilic silane compound layer with less surface irregularities. This is expected to provide an improved ability to capture specific cells such as cancer cells. Further, since the hydrophilic silane compound layer is firmly bonded to the hydrophilized surface of the substrate, it can hardly dissolve in solvents such as organic solvents, if used, for example, to wash the surface. Thus, its dissolution can be sufficiently reduced.

### DESCRIPTION OF EMBODIMENTS

The hydrophilic substrate of the present invention includes a substrate having a hydrophilized surface and a hydrophilic silane compound layer provided on at least part (part or all) of the hydrophilized surface of the substrate. By forming a hydrophilic silane compound layer on a previously hydrophilized surface of a substrate, it is possible to provide a hydrophilic substrate coated with a highly smooth hydrophilic silane compound layer having controlled surface irregularities and a small surface roughness.

Since the number of tumor cells (e.g., cancer cells) appearing in biological fluid, such as circulating tumor cells (several to hundreds of cells/1 mL of blood), is very small, it is considered important to capture as many tumor cells present in the sampled biological fluid as possible to analyze them. According to the present invention, a hydrophilized surface of a substrate may be coated with a hydrophilic silane compound to form a highly smooth hydrophilic silane compound layer having controlled surface irregularities and a small surface roughness. As the surface irregularities of the hydrophilic silane compound layer affects the ability to capture specific cells such as cancer cells, the highly smooth surface with controlled surface irregularities is expected to provide an excellent ability to capture specific cells. Thus, it is expected that by counting the number of tumor cells captured onto the hydrophilic silane compound layer on the hydrophilized substrate surface, one can determine the number of tumor cells in biological fluid, e.g., in order to evaluate the cancer-treating effect. Moreover, the captured tumor cells may be cultured and then used to determine the effects of drugs such as anticancer drugs. This allows one to determine the effects of drugs such as anticancer drugs ex vivo before administration, and also helps to screen drugs such as anticancer drugs.

The hydrophilized substrate (hydrophilized substrate surface) can be prepared by hydrophilizing a surface of a substrate. Examples of the substrate include glass such as soda-lime glass and borosilicate glass, acrylic resins (polyacrylic resins) such as polymethyl acrylate, polymethyl methacrylate, polyacrylic acid, and polymethacrylic acid; cycloolefin resins

(polycycloolefins); carbonate resins (polycarbonates); styrene resins (polystyrenes); polyester resins such as polyethylene terephthalate (PET); and polydimethylsiloxanes. The substrate may also be a metal substrate. Examples of the metal material of the metal substrate include metals such as stainless steel, nickel-titanium alloys, iron, titanium, aluminum, tin, and zinc-tungsten alloys. From the standpoint of biocompatibility, stainless steel or nickel-titanium alloys are preferred among these.

The hydrophilization may be carried out by any method capable of hydrophilizing a surface of a substrate, and examples of such methods include alkaline solution treatments, ultraviolet irradiation treatments, electron beam irradiation treatments, ion irradiation treatments, and plasma treatments. From the standpoint of controlling the surface irregularities of the hydrophilic silane compound layer, alkaline solution treatments or ultraviolet irradiation treatments are preferred among these.

Examples of alkali compounds that may be used in the alkaline solution treatments (surface treatments with alkaline solutions) include inorganic alkali compounds such as alkali metal hydroxides, alkaline-earth metal hydroxides, and ammonia water and organic alkali compounds such as tetramethylammonium hydroxide. From the standpoint of controlling the surface irregularities of the hydrophilic silane compound layer, inorganic alkali compounds are preferred among these, with alkali metal hydroxides being more preferred. Examples of the alkali metal hydroxides include sodium hydroxide, potassium hydroxide, and lithium hydroxide. Examples of the alkaline-earth metal hydroxides include magnesium hydroxide, calcium hydroxide, and barium hydroxide. These alkali compounds may be used alone or in combinations of two or more.

Examples of the alkaline solutions (liquids in which alkali compounds are dissolved) include aqueous solutions and organic solutions of alkali compounds. Aqueous solutions of alkali compounds (alkaline aqueous solutions) are preferred among these. The concentration of the alkaline solution used may be chosen appropriately according to treatment efficiency, the surface irregularities of the hydrophilic silane compound layer, and other factors. For example, the concentration of the solution may be 0.01 to 5 M (mol/L). The lower limit of the concentration is preferably 0.05 M or higher, more preferably 0.10 M or higher. The upper limit thereof is preferably 4 M or lower, more preferably 3 M or lower.

The surface treatment with the alkaline solution may be performed by any appropriate method capable of contacting the alkaline solution with the substrate surface, such as by immersion in the alkaline solution, application using, e.g., a brush, or spraying. The temperature and time of the alkaline solution treatment may be chosen appropriately according to the surface irregularities of the hydrophilic silane compound layer, treatment efficiency, and other factors. For example, the treatment temperature is suitably 5 to 80°C, and the treatment time is suitably 1 second to 6 hours.

From the standpoint of the surface irregularities of the hydrophilic silane compound layer, the alkaline solution treatment is preferably performed while agitating the alkaline solution by stirring, shaking, or other similar means. The alkaline solution treatment may be performed once or multiple times. The number of treatments may be chosen appropriately according to the surface irregularities of the hydrophilic silane compound layer and other factors.

After the alkaline solution treatment, the alkaline solution may optionally be removed from the substrate surface, e.g., by rinsing with water, high pressure washing, or scrubbing, followed by drying and other processes.

Ultraviolet irradiation treatments may be performed using known ultraviolet irradiation systems. Examples of such systems include those equipped with light sources, lamp fittings, power sources, cooling devices, conveying devices, and other components. Examples of the light sources include mercury lamps, metal halide lamps, potassium lamps, mercury xenon lamps, and flash lamps. The light source wavelength and ultraviolet irradiation conditions are not limited and may be selected as appropriate.

Electron beam irradiation treatments refer to general treatments of electron irradiation, and examples include gamma irradiation treatments and discharge phenomena such as arc discharge and corona discharge. The accelerating voltage used may be appropriately selected from the standpoint of damage to the substrate, for example.

Examples of the ion irradiation treatments include hydrogen ion irradiation, argon ion irradiation, and other known methods. Examples of the plasma treatments include hydrogen plasma irradiation, argon plasma irradiation, and other known methods. The irradiation conditions may be appropriately selected from the standpoint of damage to the substrate, for example.

At least part (part or all) of the hydrophilized substrate surface (before the formation of a hydrophilic silane compound layer) preferably has a contact angle of water of 50° or less, more preferably 40° or less, still more preferably 30° or less. The lower limit of the contact angle is not critical, and a smaller contact angle is better.

Herein, the contact angle of water can be measured as described later in EXAMPLES.

The hydrophilic substrate includes a substrate having a hydrophilized surface and a hydrophilic silane compound layer provided on at least part (part or all) of the hydrophilized surface of the substrate. The hydrophilic substrate may include a primer layer provided between the substrate surface and the hydrophilic silane compound layer. The primer layer may suitably be, for example, a layer formed from an alkoxy group-containing silane compound other than the hydrophilic silane compound, which will be described later. The number of carbon atoms of the alkoxy group is preferably 1 to 22, more preferably 1 to 16. The alkoxy group is preferably at least one selected from the group consisting of a methoxy group, an ethoxy group, a propoxy group, and a butoxy group. The alkoxy group-containing silane compound more preferably contains an ethoxy group and/or a butoxy group, still more preferably an ethoxy group and a butoxy group, among these. The alkoxy group-containing silane compound may contain a hydrocarbon group such as an alkyl group. The number of carbon atoms of the alkyl group is preferably 1 to 6, more preferably 1 to 4. Examples of the alkyl group include C1-C8 alkyl groups such as a methyl group, an ethyl group, a n-propyl group, an isopropyl group, a n-butyl group, an isobutyl group, a sec-butyl group, and a tert-butyl group.

Examples of the silane compound for forming the primer layer include monoalkoxysilanes having one alkoxy group, dialkoxysilanes having two alkoxy groups, trialkoxysilanes having three alkoxy groups, and tetraalkoxysilanes having four alkoxy groups. The silane compound may be, for example, a compound represented by the following formula (1):

R¹¹ₖSi(OR¹²)₄₋ₖ (1)

wherein each R¹¹ or each R¹² independently represents a C1-C8 saturated or unsaturated hydrocarbon group, and R¹¹ and
R¹² may be the same as or different from each other; and k represents an integer of 0 to 3.

Among these compounds, compounds having two or more alkoxy groups are preferred, with compounds having three or more alkoxy groups being more preferred.

R¹¹ and R¹² in formula (1) each preferably have 1 to 6 carbon atoms, more preferably 1 to 4 carbon atoms. R¹¹ and R¹² are each preferably a saturated hydrocarbon group.

Specific examples of the monoalkoxysilanes include trimethylmethoxysilane, trimethylethoxysilane, trimethylpropoxysilane, triethylmethoxysilane, triethylethoxysilane, and tripropylpropoxysilane. Specific examples of the dialkoxysilanes include dimethyldimethoxysilane, dimethyldiethoxysilane, dimethyldipropoxysilane, diethyldimethoxysilane, diethyldiethoxysilane, methylethyldimethoxysilane, methylethyldiethoxysilane, and dipropyldimethoxysilane. Specific examples of the trialkoxysilanes include methyltrimethoxysilane, methyltriethoxysilane, methyltripropoxysilane, ethyltrimethoxysilane, ethyltriethoxysilane, propyltrimethoxysilane, propyltriethoxysilane, and butyltrimethoxysilane. Specific examples of the tetraalkoxysilanes include tetramethoxysilane, tetraethoxysilane, tetrapropoxysilane, tetrabutoxysilane, dibutoxydiethoxysilane, butoxytriethoxysilane, and ethoxytriethoxysilane.

The silane compound for forming the primer layer may also suitably be a compound represented by the following formula (2):

(R²¹O)ₘSi(OR²²)ₙ (2)

wherein R²¹ and R²² are the same or different and each represent a C1-C8 saturated or unsaturated hydrocarbon group; and m + n = 4 with m ≥ n ≥ 0 on average.

R²¹ and R²² in formula (2) each preferably have 1 to 6 carbon atoms, more preferably 1 to 4 carbon atoms. R²¹ and R²² are each preferably a saturated hydrocarbon group.

Examples of the silane compound of formula (2) include a butoxy/ethoxy tetraalkoxysilane ("Primer coat PC-3B" available from Fluoro Technology) represented by the following formula (2-1):

(H₃C-CH₂-CH₂-CH₂-O)ₘ-Si-(O-CH₂-CH₃)ₙ (2-1)

wherein m + n = 4 with m > n > 0 on average.

The primer layer may be formed by any method capable of contacting the hydrophilized substrate surface with the silane compound for forming the primer layer. For example, the silane compound or a solution thereof may be applied or sprayed onto the hydrophilized substrate surface, or the hydrophilized substrate may be immersed in the silane compound or a solution thereof. After the treatment (formation), washing, drying, and/or other appropriate processes may be performed by known methods. The solution of the silane compound may be prepared using a solvent, concentration, and other conditions appropriately selected depending on the type of the silane compound.

In the hydrophilic substrate, a hydrophilic silane compound layer is provided on at least part (part or all) of the hydrophilized surface of the substrate, optionally with a primer layer being provided therebetween. The hydrophilic silane compound for forming the hydrophilic silane compound layer may be an appropriately selected hydrophilic silane compound. The hydrophilic silane compound may be a single compound or a combination of two or more compounds.

The hydrophilic silane compound for forming the hydrophilic silane compound layer may be an appropriately hydrophilic silane compound, but from standpoints such as the surface irregularities of the hydrophilic silane compound layer, it is preferably, for example, a polyalkylene oxide group-containing hydrophilic silane compound. Suitable examples include [alkoxy(polyalkyleneoxy)alkyl]trialkoxysilanes such as [methoxy(polyethyleneoxy)propyl]trimethoxysilane.

From standpoints such as the surface irregularities of the hydrophilic silane compound layer, the polyalkylene oxide group-containing hydrophilic silane compound may suitably be, for example, a compound represented by the formula (I) below. When the compound of formula (I) is used, the hydrophilic silane compound may suitably be a single compound of formula (I) or a combination of two or more compounds of formula (I), or a mixture of one or two or more compounds of formula (I) and one or two or more additional silane compounds.

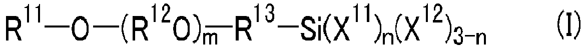

In the formula, R¹¹ represents a monovalent hydrocarbon group; R¹² and R¹³ are the same or different and each represent a divalent hydrocarbon group; each X¹¹ independently represents an alkoxy group; each X¹² independently represents a monovalent hydrocarbon group or a halogen group; m represents 1 to 50; and n represent 0 to 3.

In formula (I), the monovalent hydrocarbon group as R¹¹ may be, for example, a linear, cyclic, or branched alkyl, alkenyl, aryl, or aralkyl, particularly preferably alkyl group. The monovalent hydrocarbon group may be substituted or unsubstituted. The number of carbon atoms of R¹¹ in formula (I) is preferably 1 to 18, more preferably 1 to 10, still more preferably 1 to 4. Specific examples of the monovalent hydrocarbon group as R¹¹ include substituted or unsubstituted methyl, ethyl, n-propyl, isopropyl, n-butyl, isobutyl, sec-butyl, and tert-butyl groups.

The divalent hydrocarbon group as R¹² or R¹³ in formula (I) may be a linear, cyclic, or branched group, particularly preferably a linear group. The divalent hydrocarbon group may be substituted or unsubstituted. Specific examples of the divalent hydrocarbon group as R¹² in formula (I) include substituted or unsubstituted C1-C30 alkylene, C2-C30 alkenylene, C5-C30 cycloalkylene, C6-C30 cycloalkylalkylene, C6-C30 arylene, and C7-C30 aralkylene groups. Among these, R¹² is preferably a substituted or unsubstituted C1-C18 alkylene, more preferably C1-C10 alkylene, still more preferably C1-C6 alkylene group. R¹³ is preferably a substituted or unsubstituted C1-C20 alkylene, more preferably C1-C14 alkylene, still more preferably C1-C12 alkylene group. Specific examples of R¹² or R¹³ include substituted or unsubstituted methylene, ethylene, trimethylene, tetramethylene, pentamethylene, and hexamethylene groups.

In formula (I), the alkoxy group as X¹¹ may be a branched or unbranched group. The number of carbon atoms of the alkoxy group in formula (I) is preferably 1 to 12, more preferably 1 to 8, still more preferably 1 to 5. Specific examples of the alkoxy group include methoxy, ethoxy, n-propoxy, isopropoxy, n-butoxy, iso-butoxy, sec-butoxy, tert-butoxy, pentyloxy, hexyloxy, heptyloxy, 2-ethylhexyloxy, octyloxy, and nonyloxyl groups.

Examples of the monovalent hydrocarbon group as X¹² in formula (I) include those mentioned for the monovalent hydrocarbon group as R¹¹. Examples of the halogen group (halogen atom) as R¹² include fluorine, chlorine, and bromine.

In formula (I), m is preferably 1 to 40, more preferably 1 to 30, still more preferably 1 to 25.

In formula (I), n is preferably 1 to 3, more preferably 2 to 3.

In formula (I), R¹¹, R¹², R¹³, X¹¹, and X¹² each may be substituted with any substituent, such as a hydroxyl group and other functional groups.

For example, the hydrophilic silane compound of formula (I) may suitably be a hydrophilic silane compound represented by the formula (I-1) below. When the compound of formula (I-1) is used, the hydrophilic silane compound may suitably be a single compound of formula (I-1) or a combination of two or more compounds of formula (I-1), or a mixture of one or two or more compounds of formula (I-1) and one or two or more additional silane compounds.

R²¹-O-(CₚH₂ₚO)ₘ-(CH₂)ₖ-Si(X¹¹)ₙ(X¹²)₃₋ₙ (I-1)

In the formula, R²¹ represents a methyl group or an ethyl group; each X¹¹ independently represents an alkoxy group; each X¹² independently represents a monovalent hydrocarbon group or a halogen group; p represents 1 to 8; m represents 1 to 50; n represents 0 to 3; and k represents 1 to 20.

In formula (1-1), X¹¹, X¹², m, and n are as described above; p is preferably 1 to 5, more preferably 1 to 3; the -CₚH₂ₚO- group may be linear or branched, particularly preferably linear; and k is preferably 1 to 16, more preferably 1 to 12.

The thickness of the hydrophilic silane compound layer (the layer formed from the hydrophilic silane compound) is preferably 1 to 50 nm, more preferably 1 to 30 nm, still more preferably 1 to 20 nm. The hydrophilic silane compound layer having such a small thickness exhibits few surface irregularities. Thus, a highly smooth layer is formed, which leads to good adhesion and adsorption of specific cells such as cancer cells and stem cells.

At least part (part or all) of the surface of the hydrophilic silane compound layer (the surface of the hydrophilic silane compound layer in the hydrophilic substrate) preferably has a contact angle of water of 80° or less, more preferably 60° or less, still more preferably 50° or less. The lower limit of the contact angle is not critical, and a smaller contact angle is better.

The surface of the hydrophilic silane compound layer (the surface of the hydrophilic silane compound layer in the hydrophilic substrate) preferably has a surface roughness Ra of 0.1 µm or less, more preferably 0.05 µm or less, still more preferably 0.025 µm or less. The lower limit of the surface roughness is not critical, and a smaller surface roughness is better.

Herein, the surface roughness Ra refers to the center-line surface roughness Ra defined in JIS B 0601-2001.

The hydrophilic silane compound layer can be formed by treating (contacting) the hydrophilized surface of the substrate prepared by hydrophilizing a substrate, with a hydrophilic silane compound. For example, the hydrophilic silane compound layer may be formed by any method capable of contacting the hydrophilized substrate surface with a hydrophilic silane compound solution and/or a hydrophilic silane compound dispersion.

Preferably, the hydrophilic silane compound layer is formed by starting the treatment with the hydrophilic silane compound solution and/or hydrophilic silane compound dispersion within three hours after the hydrophilization. For example, if a long time elapses after the hydrophilized substrate is prepared by ultraviolet irradiation treatment, the hydrophilicity deteriorates with time. For this reason, it is desirable to start the treatment with the hydrophilic silane compound within three hours. Even if the treatment cannot be started within three hours, the hydrophilicity can be maintained even after a long period of time by keeping the hydrophilized substrate in contact with a polar solvent such as water and/or an alcohol after the hydrophilization.

Specifically, a hydrophilic substrate provided with a hydrophilic silane compound layer formed from a hydrophilic silane compound (a hydrophilic substrate whose surface is entirely or partially provided with a hydrophilic silane compound layer formed of a hydrophilic silane compound) can be prepared by bringing all or part of the hydrophilized substrate surface into contact with a hydrophilic silane compound solution or dispersion prepared by dissolving or dispersing a hydrophilic silane compound in any solvent, followed by retaining and drying it for a predetermined time. Examples of such known methods include: (1) a method of dropping or injecting the hydrophilic silane compound solution or dispersion onto the hydrophilized substrate surface and retaining and drying it for a predetermined time; and (2) a method of applying (spraying) the hydrophilic silane compound solution or dispersion to the substrate surface and retaining and drying it for a predetermined time. Then, the hydrophilic substrate in which a hydrophilic silane compound layer is formed on the hydrophilized substrate surface may be combined with other components as needed, to prepare an apparatus capable of, for example, capturing, culturing, and/or testing specific cells.

The solvent, injection method, application (spraying) method, and other conditions may be conventionally known materials or methods.

The retention/drying time in the method (1) or (2) may be selected appropriately according to the size of the substrate, the type of liquid introduced, and other factors. The retention time is preferably 10 seconds to 100 hours, more preferably one minute to 60 hours, still more preferably five minutes to forty hours. The drying is preferably performed at 20 to 80°C, more preferably 20 to 60°C. Moreover, the drying may be carried out under reduced pressure. Furthermore, after the predetermined time of retention, the excess hydrophilic silane compound solution or dispersion may be discharged as appropriate before drying.

The solvent may be any solvent that can dissolve a hydrophilic silane compound and may be chosen appropriately according to the hydrophilic silane compound used. Examples include water, organic solvents, and solvent mixtures thereof. Examples of the organic solvents include alcohols such as methanol, ethanol, n-propanol, i-propanol, and methoxypropanol, ketones such as acetone and methyl ethyl ketone, tetrahydrofuran, acetonitrile, ethyl acetate, toluene, and dimethyl sulfoxide (DMSO).

After forming a hydrophilic silane compound layer on the hydrophilized surface (e.g., after contacting the hydrophilized substrate surface with the hydrophilic silane compound solution or dispersion and retaining it for a predetermined time), the resulting surface may be further retained at a humidity of 50% or higher. The retention at a humidity of 50% or higher can increase the reactivity (bonding) of the hydrophilic silane compound layer to the substrate surface. The humidity is more preferably 60% or higher, still more preferably 70% or higher. The upper limit is not critical and may be 100%.

From the standpoint of adhesion (bonding) of the hydrophilic silane compound layer to the substrate surface, the retention time and temperature during the retention at a humidity of 50% or higher may be selected as appropriate. For example, the retention time is preferably 0.5 to 60 hours, and the retention temperature is preferably 20 to 60°C.

The hydrophilic silane compound layer preferably has fibronectin adsorbed to its surface.

The adsorption of fibronectin to the surface of the hydrophilic silane compound layer results in improved adsorption (adhesion) of specific cells such as cancer cells.

Fibronectin may be adsorbed onto the hydrophilic silane compound layer by any known method, for example, by bringing the hydrophilic silane compound layer into contact with a buffer solution (e.g., phosphate buffered saline (PBS)) containing fibronectin by a known method, and leaving them at a predetermined temperature for a predetermined time, optionally followed by washing. The temperature and time may be selected as appropriate, and may be, for example, about 10 to 60°C and about 0.1 to 24 hours, respectively.

From the standpoint of adsorbing fibronectin onto the hydrophilic silane compound layer, it is suitable to use, for example, a solution or dispersion preferably having a fibronectin concentration adjusted to 0.5 to 500 µg/mL, more preferably 1 to 250 µg/mL. With a fibronectin concentration adjusted within the range indicated above, excellent capture of specific cells such as cancer cells can be achieved.

The method of manufacturing a hydrophilic substrate of the present invention includes hydrophilizing a surface of a substrate, and forming a hydrophilic silane compound layer on at least part of the hydrophilized surface. As described above, by first hydrophilizing a surface of a substrate and then forming a hydrophilic silane compound layer on the hydrophilized surface, it is possible to form a highly smooth hydrophilic silane compound layer having controlled surface irregularities and a small surface roughness.

It is expected that specific cells can be captured, for example, by bringing a sample (blood or biological fluid) into contact with the hydrophilic substrate in which a hydrophilic silane compound layer is provided on at least part of a hydrophilized surface of a substrate. Then, it is expected that by counting the number of captured specific cells, one can determine the number of specific cells in the sampled blood or biological fluid, e.g., in order to evaluate the cancer-treating effect.

### EXAMPLES

The present invention is specifically described with reference to, but not limited to, examples below.

### (Example 1)

A 1 N NaOH aqueous solution in an amount of 1 mL was injected onto the surface of a chamber slide (glass slide portion: soda-lime glass, chamber size: 20 mm × 20 mm) and allowed to stand at 23°C for one hour. Thereafter, the NaOH aqueous solution was sucked out, and the chamber slide was washed with water three times. Thus, hydrophilization was performed to prepare a hydrophilized substrate.

A 0.5% by mass solution of "SIM6492.7" (Gelest, Inc., 2-[methoxy(polyethyleneoxy)propyl]trimethoxysilane, CH₃O-(CH₂CH₂O)₆₋₉-(CH₂)₃Si(OCH₃)₃) in water/methanol (50% by mass/50% by mass) was prepared.

The prepared solution in an amount of 200 µL was injected into the hydrophilized chamber slide (hydrophilized substrate) within one hour after the hydrophilization and allowed to stand at room temperature for 20 hours, followed by standing at 40°C under high humidity conditions (humidity: 75%) for four hours. Thereafter, the chamber slide was dried in vacuum at 50°C for four hours and then washed with water and methanol. Thus, a substrate (hydrophilic substrate) provided with a hydrophilic silane compound layer was prepared.

### (Example 2)

A substrate (hydrophilic substrate) provided with a hydrophilic silane compound layer was prepared as in Example 1, except that the "SIM6492.7" (Gelest, Inc.) was changed to "SIM6492.72" (Gelest, Inc., 2-[methoxy-(polyethyleneoxy)propyl]trimethoxysilane, CH₃O-(CH₂CH₂O)₉₋₁₂-(CH₂)₃Si(OCH₃)₃).

### (Example 3)

The surface of a chamber slide (glass slide portion: soda-lime glass, chamber size: 20 mm × 20 mm) was hydrophilized by UV irradiation for 30 minutes using a UV irradiator (ORC Manufacturing Co., Ltd., HMW-615N-4, 9.6 mW/cm² low-pressure UV) to prepare a hydrophilized substrate.

A 0.5% by mass solution of "SIM6492.7" (Gelest, Inc., 2-[methoxy(polyethyleneoxy)propyl]trimethoxysilane, CH₃O-(CH₂CH₂O)₆₋₉-(CH₂)₃Si(OCH₃)₃) in water/methanol (50% by mass/50% by mass) was prepared.

The prepared solution in an amount of 200 µL was injected into the hydrophilized chamber slide (hydrophilized substrate) within one hour after the hydrophilization and allowed to stand at room temperature for 20 hours, followed by standing at 40°C under high humidity conditions (humidity: 75%) for four hours. Thereafter, the chamber slide was dried in vacuum at 50°C for four hours and then washed with water and methanol. Thus, a substrate (hydrophilic substrate) provided with a hydrophilic silane compound layer was prepared.

### (Example 4)

A substrate (hydrophilic substrate) provided with a hydrophilic silane compound layer was prepared as in Example 3, except that the "SIM6492.7" (Gelest, Inc.) was changed to "SIM6492.72" (Gelest, Inc., 2-[methoxy-(polyethyleneoxy)propyl]trimethoxysilane, CH₃O-(CH₂CH₂O)₉₋₁₂-(CH₂)₃Si(OCH₃)₃).

### (Example 5)

A substrate (hydrophilic substrate) provided with a hydrophilic silane compound layer was prepared as in Example 3, except that the "SIM6492.7" (Gelest, Inc.) was changed to "SIM6492.73" (Gelest, Inc., 2-[methoxy-(polyethyleneoxy)propyl]trimethoxysilane, CH₃O-(CH₂CH₂O)₂₁-₂₄-(CH₂)₃Si(OCH₃)₃).

### (Example 6)

A substrate (hydrophilic substrate) provided with a hydrophilic silane compound layer was prepared as in Example 3, except that the "SIM6492.7" (Gelest, Inc.) was changed to a solution of "SIM6493.4" (Gelest, Inc., methoxytriethyleneoxypropyltrimethoxysilane, CH₃O-(CH₂CH₂O)₃-(CH₂)₃Si(OCH₃)₃) in water/n-butanol (25% by mass/75% by mass).

### (Example 7)

A substrate (hydrophilic substrate) provided with a hydrophilic silane compound layer was prepared as in Example 3, except that the "SIM6492.7" (Gelest, Inc.) was changed to a solution of "SIM6493.7" (Gelest, Inc., methoxytriethyleneoxyundecyltrimethoxysilane, CH₃O-(CH₂CH₂O)₃-(CH₂)₁₁Si(OCH₃)₃) in water/n-butanol (25% by mass/75% by mass).

### (Example 8)

The surface of a chamber slide (glass slide portion: soda-lime glass, chamber size: 20 mm × 20 mm) was hydrophilized by UV irradiation for 30 minutes using a UV irradiator (ORC Manufacturing Co., Ltd., HMW-615N-4, 9.6 mW/cm² low-pressure UV) to prepare a hydrophilized substrate.

A 0.5% by mass solution of "SIM6492.7" (Gelest, Inc., 2-[methoxy(polyethyleneoxy)propyl]trimethoxysilane, CH₃O-(CH₂CH₂O)₆₉-(CH₂)₃Si(OCH₃)₃) in water/methanol (50% by mass/50% by mass) was prepared.

The prepared solution in an amount of 200 µL was injected into the hydrophilized chamber slide (hydrophilized substrate) within one hour after the hydrophilization and allowed to stand at room temperature for 20 hours, followed by standing at 40°C under high humidity conditions (humidity: 75%) for four hours. Thereafter, the chamber slide was dried in vacuum at 50°C for four hours and then washed with water and methanol. Thus, a substrate provided with a hydrophilic silane compound layer was prepared.

Further, fibronectin was adsorbed onto the surface of the hydrophilic silane compound layer. Specifically, a 200 µg/mL solution of fibronectin in a phosphate buffered saline (PBS) solution was prepared and left at 40°C for one hour, followed by washing with a PBS solution. Thus, a substrate (hydrophilic substrate) provided with a hydrophilic silane compound layer was prepared.

### (Comparative Example 1)

An (untreated) chamber slide (glass slide portion: soda-lime glass, chamber size: 20 mm × 20 mm) was used.

The substrates (hydrophilic substrates) provided with hydrophilic silane compound layers prepared in the examples and the substrate prepared in the comparative example were evaluated as described below.

### (Contact angle of water on hydrophilized substrate)

A volume of 2 µL of distilled water was dropped onto the hydrophilized substrate surface. Thirty seconds later, the contact angle was measured by the θ/2 method at room temperature.

In Examples 1 and 2, the contact angle was measured one hour after the NaOH aqueous solution treatment. In Examples 3 to 8, the contact angle was measured 10 minutes after the UV treatment. The hydrophilization effect provided by the UV treatment decreased with time, and the contact angle measured three hours after the UV treatment was greater than 10°.

### (Contact angle of water on hydrophilic substrate)

A volume of 2 µL of distilled water was dropped onto the surface of the hydrophilic substrate (the surface of the hydrophilic silane compound layer). Thirty seconds later, the contact angle was measured by the θ/2 method at room temperature.

### (Thickness of hydrophilic silane compound layer (coating layer))

The thickness of the hydrophilic silane compound layer of the hydrophilic substrate was determined by measuring (photographing) a cross section of the hydrophilic silane compound layer using a TEM at an accelerating voltage of 15 kV and a magnification of 1000 times.

### (Surface roughness Ra)

The surface roughness of each hydrophilic substrate (hydrophilic silane compound layer) was measured contactless at four points using a laser microscope. The average of the four Ra values was taken as the surface roughness Ra (the average of the center-line surface roughnesses Ra defined in JIS B 0601-2001).

**[Table 1]**

| | Example 1 | Example 2 | Example 3 | Example 4 | Example 5 | Example 6 | Example 7 | Example 8 | Comparative Example 1 |
|---|---|---|---|---|---|---|---|---|---|
| Contact angle of water on hydrophilized substrate (°) | 35.4 | 35.3 | 5.2 | 5.3 | 5.3 | 5.5 | 5.3 | 5.2 | 62.5 |
| Contact angle of water on hydrophilic substrate (°) | 42.1 | 40.5 | 40.1 | 39.4 | 35.2 | 53.7 | 76.0 | 34.6 | 57.2 |
| Thickness of hydrophilic silane compound layer (nm) | 4 | 5 | 5 | 6 | 10 | 3 | 13 | 15 | 0 |
| Surface roughness (Ra) (*µ*m) | 0.008 | 0.008 | 0.005 | 0.005 | 0.004 | 0.005 | 0.004 | 0.005 | 0.003 |

The contact angle of water on each hydrophilized substrate was low, and thus the hydrophilic silane compound was sufficiently reacted with and fixed on the surface thereof. The substrates (hydrophilized substrates) of Examples 1 to 8 provided with hydrophilic silane compound layers were as colorless and transparent as the substrate of Comparative Example 1. Thus, they were considered to have a smooth surface. In fact, like the comparative example, the examples also exhibited a surface roughness Ra of smaller than 0.01 µm, indicating a sufficient smoothness. Moreover, the hydrophilic silane compound layer of each example was firmly bonded to the hydrophilized substrate surface so that it could hardly dissolve in solvents such as aqueous or organic solvents, if used, for example, to wash the surface. Thus, unreacted products and the like were sufficiently removed from the surface by washing.

## Claims

1. A hydrophilic substrate, comprising:
a substrate having a hydrophilized surface; and
a hydrophilic silane compound layer provided on at least part of the hydrophilized surface of the substrate.

2. The hydrophilic substrate according to claim 1,
wherein the hydrophilization is an alkaline solution treatment.

3. The hydrophilic substrate according to claim 1,
wherein the hydrophilization is at least one selected from the group consisting of ultraviolet irradiation, electron beam irradiation, ion irradiation, and plasma treatments.

4. The hydrophilic substrate according to any one of claims 1 to 3,
wherein the hydrophilic silane compound layer is formed from at least one of a hydrophilic silane compound solution or a hydrophilic silane compound dispersion.

5. The hydrophilic substrate according to claim 4,
wherein the hydrophilic silane compound layer is formed by starting treatment with the at least one of a hydrophilic silane compound solution or a hydrophilic silane compound dispersion within three hours after the hydrophilization.

6. The hydrophilic substrate according to any one of claims 1 to 5,
wherein the hydrophilic silane compound layer is formed from a hydrophilic silane compound represented by the following formula (I): 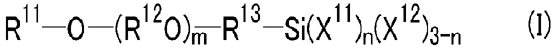 wherein R¹¹ represents a monovalent hydrocarbon group; R¹² and R¹³ are the same or different and each represent a divalent hydrocarbon group; each X¹¹ independently represents an alkoxy group; each X¹² independently represents a monovalent hydrocarbon group or a halogen group; m represents 1 to 50; and n represents 0 to 3.

7. The hydrophilic substrate according to claim 6,
wherein the silane compound of formula (I) is a compound represented by the following formula (I-1):
R²¹-O-(CₚH₂ₚO)ₘ-(CH₂)ₖ-Si(X¹¹)ₙ(X¹²)₃₋ₙ (I-1)
wherein R²¹ represents a methyl group or an ethyl group;
each X¹¹ independently represents an alkoxy group; each X¹² independently represents a monovalent hydrocarbon group or
a halogen group; p represents 1 to 8; m represents 1 to 50;
n represents 0 to 3; and k represents 1 to 20.

8. The hydrophilic substrate according to any one of claims 1 to 7,
wherein the hydrophilic silane compound layer has fibronectin adsorbed thereto.

9. The hydrophilic substrate according to any one of claims 1 to 8,
wherein the hydrophilic silane compound layer has a thickness of 1 to 50 nm.

10. A method of manufacturing a hydrophilic substrate, comprising:
hydrophilizing a surface of a substrate; and
forming a hydrophilic silane compound layer on at least part of the hydrophilized surface.

11. The method of manufacturing a hydrophilic substrate according to claim 10,
wherein the hydrophilization is an alkaline solution treatment.

12. The method of manufacturing a hydrophilic substrate according to claim 10,
wherein the hydrophilization is at least one selected from the group consisting of ultraviolet irradiation, electron beam irradiation, ion irradiation, and plasma treatments.

13. The method of manufacturing a hydrophilic substrate according to any one of claims 10 to 12,
wherein the hydrophilic silane compound layer is formed from at least one of a hydrophilic silane compound solution or a hydrophilic silane compound dispersion.

14. The method of manufacturing a hydrophilic substrate according to claim 13,
wherein the hydrophilic silane compound layer is formed by starting treatment with the at least one of a hydrophilic silane compound solution or a hydrophilic silane compound dispersion within three hours after the hydrophilization.

15. The method of manufacturing a hydrophilic substrate according to any one of claims 10 to 14,
wherein the hydrophilic silane compound layer is formed from a hydrophilic silane compound represented by the following formula (I): 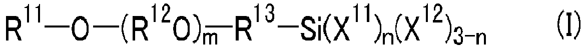 wherein R¹¹ represents a monovalent hydrocarbon group; R¹² and R¹³ are the same or different and each represent a divalent hydrocarbon group; each X¹¹ independently represents an alkoxy group; each X¹² independently represents a monovalent hydrocarbon group or a halogen group; m represents 1 to 50; and n represents 0 to 3.

16. The method of manufacturing a hydrophilic substrate according to any one of claims 10 to 15,
wherein the hydrophilic silane compound layer has a thickness of 1 to 50 nm.
